# EUROPEAN PATENT SPECIFICATION

(11) **EP 3 282 982 B1**
(45) Date of publication and mention of the grant of the patent: **10.04.2019**
(21) Application number: 16724112.4
(22) Date of filing: 12.04.2016
(51) Int. Cl.: A61B 17/70

(54) **EXPANDABLE POLYAXIAL SPINAL SYSTEM**
EXPANDIERBARES POLYAXIALES WIRBELSÄULENSYSTEM
SYSTÈME VERTÉBRAL POLYAXIAL EXTENSIBLE

(30) Priority: 17.04.2015 US 201562178691 P
(43) Date of publication of application: 21.02.2018
(73) Proprietor: Apifix Ltd., 2161401 Carmiel (IL)
(72) Inventor: ARNIN, Uri, 3603117 Kiryat Tivon (IL)
(74) Representative: White, Duncan Rohan
(86) International application number: PCT/IB2016/052075
(87) International publication number: WO 2016/166663

(56) References cited:
- WO-A2-2004/075778
- US-A1- 2005 182 401
- US-A1- 2012 130 430

## Description

### FIELD OF THE INVENTION

The present invention relates generally to spinal implants and prostheses, and particularly to a spinal pedicle screws based system designed to be expandable and maintain polyaxial motion in situ.

### BACKGROUND OF THE INVENTION

Spine scoliosis is affecting significant portion of the population. Current surgical treatment involves many times the use of an intervertebral pedicle screws based systems designed to be placed between two or more vertebrae.

The use of standard pedicle screws system is typically associated with spinal fusion, which has some known negative impacts. It is of value therefor to have a non-fusion system that can prevent or correct scoliosis of some patients.

WO 2004075778 A2 discloses a low-profile surgical rod implant device that allows the length of a rod spanning two bone screws to be adjusted at the time of implantation

US 2012130430 A1 discloses an extra-discal device for intervertebral stabilization which includes at least one rigid part connected to pedicle screws by an element.

US 2005182401 A1 discloses spinal stabilization devices, systems and methods that include at least one pedicle screw and at least one mechanism that supports three degrees of rotational freedom relative to the pedicle screw.

### SUMMARY OF THE INVENTION

The present invention seeks to provide an improved spinal pedicle screws based system that can be expanded after the screws are inserted to the pedicles of the two vertebrae and maintain spinal motion via one or more polyaxial joints.

There is thus provided in accordance with a non-limiting embodiment of the present invention a spinal system including a first distraction rod which has a first connector mounted at an end thereof, and a second distraction rod which has a second connector mounted at an end thereof, both of the rods being arranged to move in a housing, and wherein each of the connectors is mounted on a polyaxial joint and secured by a fastener, each of the polyaxial joints being attached to, or part of, a pedicle screw, and wherein the fastener is tightened against a portion of the polyaxial joint but does not inhibit polyaxial movement of the polyaxial joint, such that even after tightening the fastener, the connectors cannot move off the polyaxial joints but the polyaxial joints are free to move polyaxially at all times.

In accordance with an embodiment of the present invention at least one of the first and second distraction rods is affixed by a distraction fastener at any desired location along the housing.

In accordance with an embodiment of the present invention at least one of the first and second distraction rods is rotated about its longitudinal axis before being locked in place by the distraction fastener.

In accordance with an embodiment of the present invention at least one of the distraction rods is at least partially threaded and mates with an internal thread in the housing.

In accordance with an embodiment of the present invention both of the distraction rods are at least partially threaded and mate with an internal thread in the housing, and wherein threads of one of the distraction rods are right-handed and threads of the other distraction rod are left-handed.

In accordance with an embodiment of the present invention the housing includes a fluid inlet for introducing therethrough fluid.

In accordance with an embodiment of the present invention the housing includes a biasing device operative to apply a biasing force on at least one of the first and second distraction rods.

### BRIEF DESCRIPTION OF THE DRAWINGS

The present invention will be understood and appreciated more fully from the following detailed description taken in conjunction with the drawings in which:
Fig. 1 is a simplified pictorial illustration of a spinal system, in an initial contracted configuration before distraction, constructed and operative in accordance with a non-limiting embodiment of the invention;
Fig. 2 is a simplified pictorial illustration of the spinal system in an expanded/distracted configuration;
Fig. 3 is a simplified, cutaway illustration of the system in its expanded/distracted configuration;
Fig. 4 is a simplified pictorial illustration of a spinal system, constructed and operative in accordance with another non-limiting embodiment of the invention, wherein distraction is done using threaded elements;
Fig. 5 is a simplified pictorial illustration of a spinal system, constructed and operative in accordance with another non-limiting embodiment of the invention, wherein distraction is done using hydraulic or pneumatic force; and
Fig. 6 is a simplified pictorial illustration of a spinal system, constructed and operative in accordance with another non-limiting embodiment of the invention, wherein distraction is done using a biasing device.

### DETAILED DESCRIPTION OF EMBODIMENTS

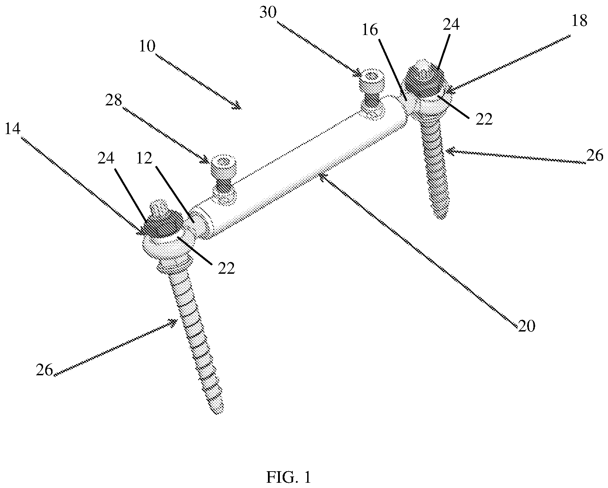

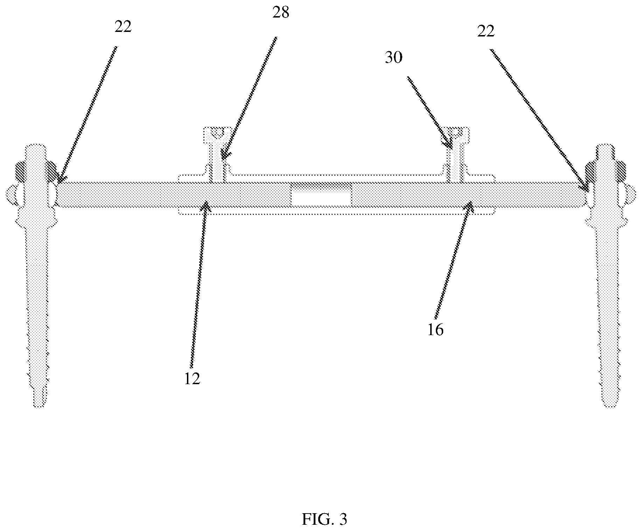

Reference is now made to Figs. 1-3, which illustrate a spinal system 100, constructed and operative in accordance with a non-limiting embodiment of the invention.

The spinal system 10 includes a first distraction rod 12 which has a first connector 14 mounted at an end thereof, and a second distraction rod 16 which has a second connector 18 mounted at an end thereof. Both rods 12 and 16 are arranged to move (e.g., translate or slide) in a housing 20. Both connectors 14 and 18 may each include a ring mounted on a polyaxial joint 22 (which may be, without limitation, a spherically shaped head) and secured by a fastener 24, such as a nut. The polyaxial joint 22 may be attached to, or may be part of, a pedicle screw 26. The fastener 24 is tightened against a portion (in the illustration, the upper portion) of the polyaxial joint 22 but does not inhibit polyaxial movement of the polyaxial joint. Thus, even after tightening the fastener 24, the connector 14 or 18 cannot move off the polyaxial joint but the polyaxial joint is free to move polyaxially at all times.

Distraction fasteners 28 and 30 may fasten the first and second distraction rods 12 and 16, respectively, at any desired location along housing 20.

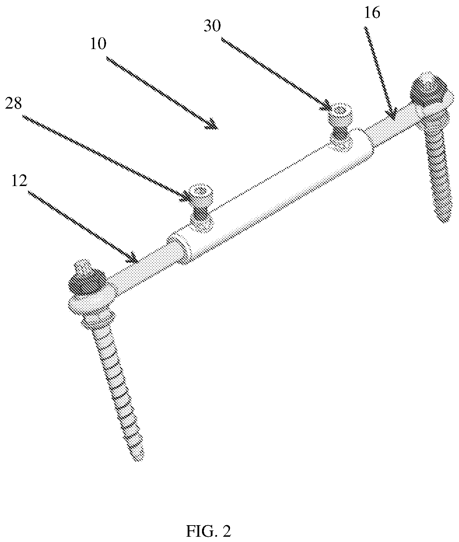

After inserting the pedicle screws 26 into the patient's vertebrae it is possible to distract or expand (the terms being used interchangeably) the distance between the screws by using a distractor (not shown) or any other suitable means. The distracted orientation is shown in Fig. 2. After distraction the distraction fasteners 28 and 30 can be used to fix the distance between the pedicle screws.

As seen in Figs. 1 and 2, the first and second distraction rods 12 and 16 may be rotated about their respective longitudinal axes before being locked in place by distraction fasteners 28 and 30. For example, only one of the distraction rods may be rotated while the other is not rotated, or both may be rotated. As another example, only one of the distraction rods may be distracted while the other is not distracted, or both may be distracted. Whichever rods is not distracted may be locked in place by the distraction fastener, or alternatively, may be left unlocked, in which case that rod is free to move in translation and in rotation.

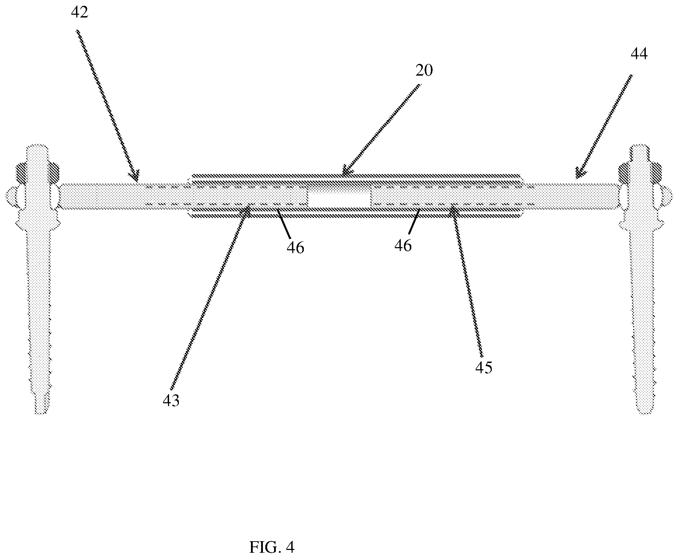

Reference is now made to Fig. 4, which illustrates a spinal system, constructed and operative in accordance with another non-limiting embodiment of the invention, with like elements being designated by like numerals. In this embodiment, distraction rods 42 and 44 are at least partially threaded and mate with an internal thread 46 in housing 20. Distraction may be done by rotating the rods with respect to the housing, that is, by rotating the rods with the housing stationary or by rotating the housing with the rods not rotating, or by rotating both the rods and the housing. In one embodiment, threads 43 of rod 42 are right-hand threads whereas threads 45 of rod 44 are left-hand threads. In this manner, rotating the housing in one direction causes the rods 42 and 44 to move simultaneously in opposite directions. After distraction to the desired distance between the pedicle screws, distraction fasteners (not shown) may be used to fix the system at the desired position.

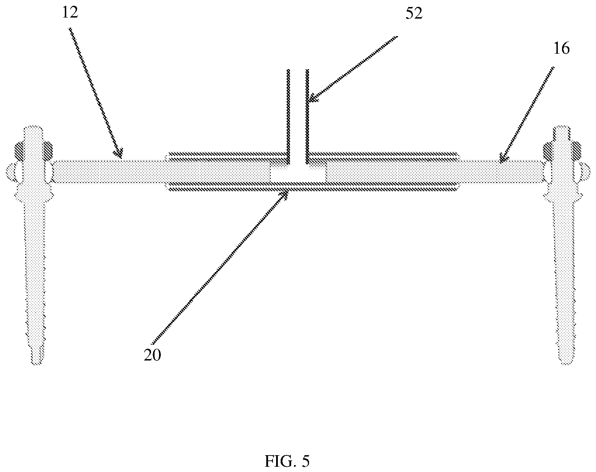

Reference is now made to Fig. 5, which illustrates a spinal system, constructed and operative in accordance with another non-limiting embodiment of the invention, with like elements being designated by like numerals. In this embodiment, distraction is done using hydraulic or pneumatic force. For example, housing 20 may be provided with a fluid inlet 52 for introducing therethrough fluid (e.g., water, saline, air, etc.). The fluid pressure acts on rods 12 and 16 and increase the distance between them. Sealing rings (not shown) may be provided to seal the fluid in the housing 20. The fluid may be introduced at any point during or after the surgical procedure.

In one embodiment, one of the rods can be affixed to the housing while the other rod is distracted by the fluid (hydraulic or pneumatic) force.

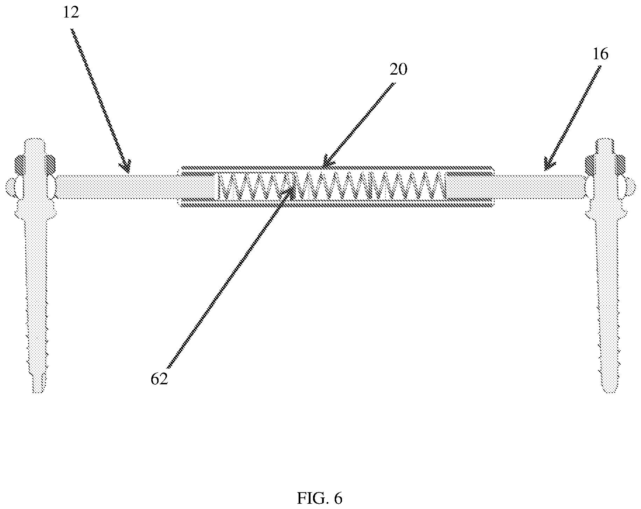

Reference is now made to Fig. 6, which illustrates a spinal system, constructed and operative in accordance with another non-limiting embodiment of the invention, with like elements being designated by like numerals. In this embodiment, distraction is done using a biasing device 62 disposed in the housing, such as a coil spring or flexible band. Biasing device 62 may be made of metal or elastomeric materials. Biasing device 62 may apply a constant or variable spring force on the rods 12 and 16.

In one embodiment, one of the rods can be affixed to the housing while the other rod is distracted by the spring force.

## Claims

1. A spinal system (10) comprising:
a first distraction rod (12) which has a first connector (14) mounted at an end thereof; and
**characterised by** a second distraction rod (16) which has a second connector (18) mounted at an end thereof, both of said rods (12, 16) being arranged to move in a housing (20), and wherein each of said connectors (14, 18) is mounted on a polyaxial joint (22) and secured by a fastener (24), each of said polyaxial joints being attached to, or part of, a pedicle screw (26), and wherein said fastener (24) is tightened against a portion of said polyaxial joint (22) but does not inhibit polyaxial movement of said polyaxial joint (22), such that even after tightening said fastener (24), said connectors (14, 18) cannot move off said polyaxial joints (22) but said polyaxial joints (22) are free to move polyaxially at all times.

2. The spinal system (10) according to claim 1, wherein at least one of said first and second distraction rods (12, 16) is affixed by a distraction fastener (28, 30) at any desired location along said housing (20).

3. The spinal system (10) according to claim 2, wherein at least one of said first and second distraction rods (12, 16) is rotated about its longitudinal axis before being locked in place by said distraction fastener (28, 30).

4. The spinal system (10) according to claim 1, wherein at least one of said distraction rods (42, 44) is at least partially threaded and mates with an internal thread (46) in said housing (20).

5. The spinal system (10) according to claim 1, wherein both of said distraction rods (42, 44) are at least partially threaded and mate with an internal thread (46) in said housing (20), and wherein threads of one of said distraction rods (42, 44) are right-handed and threads of the other distraction rod (44, 42) are left-handed .

6. The spinal system (10) according to claim 1, wherein said housing (20) comprises a fluid inlet (52) for introducing therethrough fluid.

7. The spinal system (10) according to claim 1, wherein said housing (20) comprises a biasing device (62) operative to apply a biasing force on at least one of said first and second distraction rods (12, 16).

## Patentansprüche

1. Wirbelsäulensystem (10), umfassend:
eine erste Distraktionsstange (12), welche einen ersten Verbinder (14) aufweist, welcher an einem Ende derselben montiert ist;
und
**gekennzeichnet durch** eine zweite Distraktionsstange (16), welche einen zweiten Verbinder (18) aufweist, welcher an einem Ende derselben montiert ist, wobei beide Stangen (12, 16) angeordnet sind, um sich innerhalb eines Gehäuses (20) zu bewegen, und wobei jeder der Verbinder (14, 18) auf einem polyaxialen Gelenk (22) montiert ist und durch ein Befestigungselement (24) befestigt ist, wobei jeder der polyaxialen Gelenke an eine oder an einen Teil einer Pedikelschraube (26) angeschlossen ist und wobei das Befestigungselement (24) gegen einen Abschnitt des polyaxialen Gelenks (22) gespannt ist, ohne die polyaxiale Bewegung des polyaxialen Gelenks (22) zu verhindern, sodass auch nach dem Spannen des Befestigungselements (24), die Verbinder (14, 18) sich nicht von den polyaxialen Gelenken (22) weg bewegen können, während die polyaxialen Gelenke (22) jederzeit polyaxial frei beweglich sind.

2. Wirbelsäulensystem (10) nach Anspruch 1, wobei mindestens eine der ersten und zweiten Distraktionsstange (12, 16) mittels eines Distraktionsbefestigungselements (28, 30) an jeder gewünschten Stelle entlang dem Gehäuse (20) befestigt ist.

3. Wirbelsäulensystem (10) nach Anspruch 2, wobei mindestens eine der ersten und zweiten Distraktionsstange (12, 16) um ihre Längsachse gedreht wird, bevor sie in ihrer Position durch das Distraktionsbefestigungselement (28, 30) verriegelt wird.

4. Wirbelsäulensystem (10) nach Anspruch 1, wobei mindestens eine der Distraktionsstangen (42, 44) mindestens teilweise mit einem Gewinde versehen ist und mit einem Innengewinde (46) im Gehäuse (20) eingreift.

5. Wirbelsäulensystem (10) nach Anspruch 1, wobei beide Distraktionsstangen (42, 44) mindestens teilweise mit einem Gewinde versehen sind und mit einem Innengewinde (46) im Gehäuse (20) eingreifen, und wobei Gewindegänge einer der Distraktionsstangen (42, 42) rechtshändig sind und Gewindegänge der anderen Distraktionsstange (44, 42) linkshändig sind.

6. Wirbelsäulensystem (10) nach Anspruch 1, wobei das Gehäuse (20) einen Fluideingang (52) zum Einführen eines Fluids durch denselben umfasst.

7. Wirbelsäulensystem (10) nach Anspruch 1, wobei das Gehäuse (20) eine Vorspannvorrichtung (62) umfasst, welche eine Vorspannkraft auf mindestens eine der ersten und der zweiten Distraktionsstange (12, 16) ausübt.

## Revendications

1. Système vertébral (10), comprenant :
une première tige de distraction (12) comportant un premier connecteur (14) monté sur une de ses extrémités ; et
**caractérisé par** une deuxième tige de distraction (16) comportant un deuxième connecteur (18) monté sur une de ses extrémités, lesdites deux tiges (12, 16) étant configurées pour se déplacer dans un logement (20), et dans lequel chacun desdits connecteurs (14, 18) est monté sur un joint polyaxial (22) et fixé par un élément de fixation (24), chacun desdits joints polyaxiaux étant fixé sur une vis de pédicule (24), et dans lequel ledit élément de fixation (24) est serré contre une partie dudit joint polyaxial (22) mais n'empêche pas le déplacement polyaxial dudit joint polyaxial (22), de sorte, que même après le serrage dudit élément de fixation (24), lesdits connecteurs (14, 18) ne peuvent pas se déplacer à l'écart desdits joints polyaxiaux (22), lesdits joints polyaxiaux (22) pouvant toutefois se déplacer librement de manière polyaxiale à tout moment.

2. Système vertébral (10) selon la revendication 1, dans lequel au moins une desdites première et deuxième tiges de distraction (12, 16) est fixée par une attache de distraction (28, 30) au niveau d'un quelconque emplacement voulu le long dudit logement (20).

3. Système vertébral (10) selon la revendication 2, dans lequel au moins une desdites première et deuxième tiges de distraction (12, 16) est tournée autour de son axe longitudinal avant d'être bloquée dans sa position par ladite attache de distraction (28, 30).

4. Système vertébral (10) selon la revendication 1, dans lequel au moins une desdites tiges de distraction (42, 44) est au moins partiellement filetée et s'accouple avec un filetage intérieur (46) dans ledit logement (20).

5. Système vertébral (10) selon la revendication 1, dans lequel lesdites deux tiges de distraction (42, 44) sont au moins partiellement filetées et s'accouplent avec un filetage intérieur (46) dans ledit logement (20), et dans lequel les filetages de l'une desdites tiges de distraction (42, 44) sont des filetages à droite, les filetages de l'autre tige de distraction (44, 42) étant des filetages à gauche.

6. Système vertébral (10) selon la revendication 1, dans lequel ledit logement (20) comprend une entrée de fluide (52) pour introduire un fluide à travers celui-ci.

7. Système vertébral (10) selon la revendication 1, dans lequel ledit logement (20) comprend un dispositif de sollicitation (62) servant à appliquer une force de sollicitation sur au moins une desdites première et deuxième tiges de distraction (12, 16).
